# EUROPÄISCHE PATENTSCHRIFT

(11) **EP 2 891 481 B1**
(45) Veröffentlichungstag und Bekanntmachung des Hinweises auf die Patenterteilung: **28.02.2018**
(21) Anmeldenummer: 15150248.1
(22) Anmeldetag: 07.01.2015
(51) Int. Cl.: A61H 7/00, A61F 9/08, A63B 23/04, G09B 21/00, E01C 15/00, E04F 15/02, A61H 3/06, A61H 39/04

(54) **VORRICHTUNG ZUR SENSORISCHEN VERMITTLUNG VON UMWELTEINDRÜCKEN AN EINEN BENUTZER**
Device for sensory transmission of environmental impressions to a user
Dispositif de transmission sensorielle de sensations de l'environnement à un utilisateur

(30) Priorität: 07.01.2014 DE 102014100080
(43) Veröffentlichungstag der Anmeldung: 08.07.2015
(73) Patentinhaber: Graetz, Joerg Christian, 07422 Bad Blankenburg (DE)
(72) Erfinder: Graetz, Joerg Christian, 07422 Bad Blankenburg (DE)
(74) Vertreter: Oehmke, Volker

(56) Entgegenhaltungen:
- EP-A2- 1 459 723
- DE-A1-102009 059 820
- DE-A1-102011 119 864
- JP-A- 2012 163 929
- US-A- 5 733 127

## Beschreibung

Die Erfindung betrifft eine Vorrichtung zur sensorischen Vermittlung von Umwelteindrücken an einen Benutzer in Form eines Parcours.

Solche Parcours sind in verschiedenen Ausgestaltungen, beispielsweise als Tast-, Riech- und / oder Hörparcours, bekannt. Sensorische Tastreize können dabei von einem Benutzer beispielsweise über die Füße und / oder die Hände in sogenannten Fußparcours bzw. Handparcours aufgenommen werden.

Unter einem Fußparcours wird eine begehbare Trittfläche verstanden, die üblicherweise eine vorgegebene Ausdehnung und Form aufweist. Die Trittfläche kann dabei verschiedenartige Oberflächenstrukturen aufweisen. Sehr häufig werden Fußparcours ohne Schuhwerk belaufen, um die Reize der verschiedenartigen Oberflächenstrukturen auf den jeweiligen Benutzer des Fußparcours zu intensivieren oder erst zu ermöglichen. Auf dem Fußparcours können auch Objekte aufgelegt oder befestigt sein, deren Reizwirkungen ebenfalls durch den Benutzer erfahren werden können.

Fußparcours können zur spielerischen Heranführung von Kindern, Jugendlichen und Erwachsenen an neue Sinneseindrücke, aber auch für therapeutische Zwecke genutzt werden.

Allgemein können Fußparcours dadurch beschrieben werden, dass unterschiedliche Materialien nacheinander auf einem Weg ausgelegt sind. Besonders intensiv ist die Wahrnehmung der verschiedenen Materialien, wenn ein Benutzer des Fußparcours diesen barfuß benutzt. Der Benutzer kann auch mit geschlossenen oder verbundenen Augen durch eine sehende Hilfsperson über den Fußparcours geführt werden.

Entsprechend werden bei einem Handparcours unterschiedliche Materialien und Gegenstände (fortan: Objekte) mit der Hand bzw. den Händen ertastet und deren Eigenschaften erfühlt.

Eine Anwendung eines Handparcours ist in der US 5,733,127 A offenbart. Um nicht-sehenden Benutzern von Einrichtungen wie öffentlichen Toiletten oder Parkanlagen vor dem Betreten der Einrichtungen einen Überblick über die Form der Einrichtung sowie über dort vorhandene Objekte zu geben, wird vor der Einrichtung eine Tastfläche fest angebracht. Gegebenenfalls können innerhalb der Einrichtung weitere Tastflächen fest angebracht sein, die der nicht-sehende Benutzer aber offenbar anhand seiner Erinnerung an die zuvor erfühlte Tastfläche finden muss. Eine Orientierung und eine Ermittlung seines augenblicklichen Standorts ist für den Benutzer ausschließlich aufgrund seiner Erinnerung möglich.

Es sind Fußparcours bekannt, die aus einer Aneinanderreihung von umgrenzten Bereichen, beispielsweise von Rahmen aus Holz, gebildet sind. In jedem der Rahmen ist ein anderes Material, wie beispielsweise Steine verschiedener Größe und Beschaffenheit, Sand, Erden, Streu, Gras usw., vorhanden. Es ist auch möglich, dass verschiedene Objekte auf einem Untergrund, beispielsweise einer Vliesbahn, ausgelegt sind und der Benutzer über die Vliesbahn läuft und dabei die von den Objekten hervorgerufenen Sinneseindrücke als einen Teil der rezipierten Umwelteindrücke intensiv erfährt.

In Abhängigkeit der Zielstellung der Verwendung eines Fußparcours kann ein Benutzer sehend den Fußparcours absolvieren. Eine intensivere Wahrnehmung der Sinneseindrücke kann erreicht werden, wenn der Benutzer mit geschlossenen oder verbundenen Augen über den Fußparcours läuft. Dabei ist es erforderlich, dass der Benutzer durch eine Hilfsperson oder entlang einer Hilfsstruktur, beispielsweise eines Geländers, über den Fußparcours geführt wird. Der Benutzer braucht sich dabei zwar nicht selbst zu orientieren, die Hilfsperson bzw. die Hilfsstruktur muss jedoch bereitgestellt werden. Eine zielgerichtete Bewegung eines nicht-sehenden Benutzers über den Parcours ist nicht möglich.

Mit der DE 10 2009 059 820 A1 wird ein Verfahren offengelegt nach dem einem Blinden seine Umgebung als tastbare Abbildung wahrnehmbar gemacht wird. Hierbei wird die Umgebung optisch aufgenommen und elektronisch als senkrechte Projektion dargestellt. Die Darstellung erfolgt auf einer tastbaren Oberfläche, wobei alle im Aufenthaltsfeld aus der Ebene herausragenden Objekte durch herausragende Stifte wahrgenommen werde. Die taktile Wahrnehmung ist vergleichbar zur Blindenschrift. Der Standort des Blinden im Aufenthaltsfeld wird durch Laser- oder Ultraschallmessung bestimmt und auf dem Tastfeld von einem animierten Stift angezeigt. Der Erfindung liegt die Aufgabe zugrunde, eine Vorrichtung zur sensorischen Vermittlung von Umwelteindrücken vorzuschlagen, bei der die im Stand der Technik auftretenden Einschränkungen vermieden werden.

Die Aufgabe wird gelöst durch eine Vorrichtung, die eine begehbare Trittfläche und eine handhaltbare Tastfläche aufweist, zur sensorischen Vermittlung von Umwelteindrücken an einen Benutzer und zur Ermittlung eines aktuellen Standorts des Benutzers auf der begehbaren Trittfläche durch Abgleichen von über die Fußsohlen des Benutzers erspürten Sinneseindrücken von der Trittfläche mit den Sinneseindrücken, die der Benutzer über die Hände von der handhaltbaren Tastfläche erhält, wobei die Tastfläche zur Trittfläche im geometrischen Sinne ähnlich ist. Eine Figur ist im geometrischen Sinne zu einer anderen Figur dann ähnlich, wenn beide Figuren gleiche Seiten- und Winkelverhältnisse aufweisen. Dabei sind die Figuren durch zentrische Streckung und / oder durch Kongruenzabbildungen ineinander überführbar. Auf die Trittfläche und die Tastfläche übertragen sind diese einander ähnlich, wenn deren Umrisse (nachfolgend auch als Form bezeichnet) sich so ähneln, dass diese durch einen Benutzer als offensichtlich einander entsprechend erkannt werden können. Als ähnlich im Sinne dieser Beschreibung werden auch Formen verstanden, die dem Benutzer einen im Wesentlichen gleichen Eindruck der Form vermitteln. Dabei können kleinere Abweichungen, beispielsweise eine stilisierte Abbildung der Trittfläche durch die Tastfläche, oder umgekehrt, durch den Benutzer erkannt und gewichtet werden, so dass eine Erkennung der Ähnlichkeit für den Benutzer möglich bleibt. Gleiches gilt für Proportionen von Trittfläche und Tastfläche.

Sowohl die Trittfläche als auch die Tastfläche sind durch mindestens eine Schicht eines Materials gebildet. Beide weisen vorzugsweise einen äußeren Umriss auf, so dass deren Form durch den äußeren Umriss festgelegt ist. Die Trittfläche, die Tastfläche oder beide können innere Umrisse, beispielsweise Aussparungen, aufweisen. Die Trittfläche und die Tastfläche können auch hinsichtlich der inneren Umrisse einander im oben angegebenen Sinne ähnlich sein.

Kern der Erfindung ist das Vorhandensein der Tastfläche und deren Ähnlichkeit zu der Trittfläche, wodurch für einen nicht-sehenden Benutzer der Vorrichtung die Möglichkeit eröffnet ist, ohne Hilfspersonen oder Hilfsgeräte nicht nur die Trittfläche zu belaufen, sondern sich anhand der Tastfläche zu orientieren und seinen aktuellen Standort auf der Trittfläche zu ermitteln und einen bereits zurückgelegten Weg auf der Trittfläche nachzuvollziehen und einen noch zurückzulegenden Weg selbständig zu planen.

In den erfindungsgemäßen Vorrichtung weist die Tastfläche mindestens zwei Tastflächenbereiche mit jeweils einer Oberflächenstruktur auf. Dabei können die Oberflächenstrukturen voneinander verschieden sein. In weiteren Ausgestaltungen der Vorrichtung können die Oberflächenstrukturen auch gleich sein. Dies ist beispielsweise dann möglich, wenn die Tastfläche aus mehreren Teiltastflächen zusammengesetzt ist.

Teiltastflächen können vorteilhaft vorgesehen sein, um eine aktuell zu benutzende Tastfläche aus einer Auswahl von vorhandenen Teiltastflächen zusammenzusetzen. Durch eine solche Ausgestaltung der erfindungsgemäßen Vorrichtung ist diese variabel gestaltbar.

Es ist außerdem möglich, dass auf der Tastfläche eine Anzahl von Objekten (fortan auch: Tastobjekte) an jeweiligen Positionen angeordnet sind. Die Tastobjekte können dabei mit der Tastfläche verbunden sein. Tastobjekte können dabei aufgelegt oder lösbar befestigt sein, beispielsweise durch Feststecken mittels Nadeln oder anderer Hilfsmittel oder durch Verwenden von Klettverschlüssen. Die Tastobjekte können auch an der Tastfläche angeklebt, angeheftet oder angenäht sein.

Objekte bzw. Tastobjekte sind Körper, wie beispielsweise Bälle, Wollknäuel, Stäbe oder beliebige andere Gegenstände, die auf der Trittfläche bzw. auf der Tastfläche angeordnet sind.

In der erfindungsgemäßen Vorrichtung weist die Trittfläche Trittflächenbereiche mit jeweils einer Oberflächenstruktur auf. Solche Trittflächenbereiche liegen im Wesentlichen in der Ebene der Trittfläche und können beispielsweise durch in die Trittfläche integrierte Kacheln, Stoffbahnen, Holzstücke usw. gebildet sein. Trittflächenbereiche können beispielsweise auch dadurch gebildet sein, dass die Trittfläche aus Stücken aus verschiedenen Materialien zusammengesetzt ist.

Zur verbesserten Orientierung eines Benutzers der erfindungsgemäßen Vorrichtung entsprechen die Oberflächenstrukturen der Tastflächenbereiche den Oberflächenstrukturen der Trittflächenbereiche und die Tastflächenbereiche korrespondieren hinsichtlich ihrer Lage und Ausdehnung mit den Lagen und Ausdehnungen der Trittflächenbereiche. So kann ein Benutzer . seine über die Fußsohlen erspürten Sinneseindrücke mit den Sinneseindrücken abgleichen, die er über die Hände von der Tastfläche erhält.

Außerdem ist es möglich, dass auf der Trittfläche eine Anzahl von Objekten angeordnet sind, deren jeweilige tatsächliche Objektpositionen bekannt sind, und dass die Positionen der Objekte auf der Tastfläche korrespondierende Objektpositionen sind, die mit den tatsächlichen Objektpositionen der Objekte auf der Trittfläche korrespondieren. Für Trittflächenbereiche und Tastflächenbereiche gilt das für die tatsächliche Objektposition und die korrespondierende Objektposition Gesagte entsprechend. Die Tastobjekte auf der Tastfläche können den Objekten auf der Trittfläche entsprechen. Dabei müssen die Tastobjekte und die Objekte auf der Trittfläche nicht die gleiche Größe, Form oder Anordnung aufweisen. So kann beispielsweise auf der Trittfläche eine Anzahl von Hölzern in einer unregelmäßigen Anordnung liegen, während auf der Tastfläche eine andere, aber ebenfalls unregelmäßige Anordnung, beispielsweise von Streichhölzern, vorhanden ist. Auch kann auf der Trittfläche beispielsweise ein Wollknäuel angeordnet sein, während auf der Tastfläche lediglich einige Wollfäden angebracht sind.

Auch ist es möglich, dass auf der Trittfläche eine Anzahl von Objekten angeordnet sind, deren jeweilige tatsächliche Objektpositionen bekannt sind. Es ist nicht erforderlich, dass die tatsächlichen Objektpositionen dem Benutzer der erfindungsgemäßen Vorrichtung bekannt sind. Vielmehr können die tatsächlichen Objektpositionen einer Hilfsperson bekannt sein, welche die Vorrichtung für eine Benutzung vorbereitet hat. Die Objekte können beispielsweise durch einen Benutzer zunächst mit den Füßen ertastet werden, um diese Objekte dann aufzuheben und auf der Tastfläche an den korrespondierenden Orten abzulegen.

Es ist natürlich auch möglich, dass lediglich das Vorhandensein und die Positionen von Objekten der Trittfläche über die Tastfläche vermittelt sind. So können auf der Tastfläche einheitlich gestaltete Tastobjekte vorhanden sein, während auf der Trittfläche Objekte verschiedener Eigenschaften angeordnet sind. Eine Aufgabe für den Benutzer kann dann darin bestehen, die auf der Trittfläche vorhandenen Objekte zu finden und deren Eigenschaften zu bestimmen.

Die Trittfläche kann aus mindestens einer zusammenhängenden Schicht eines oder mehrerer Materialien bestehen. Sie kann beispielsweise auf einem (Fuß-)boden befestigt oder in diesen integriert sein.

Weiterhin kann die Trittfläche auf einer Trägermatte angeordnet sein, beispielsweise aufgelegt, aufgeklebt, aufgenäht oder mit der Trägermatte mit einem Klettverschluss verbunden sein. Eine Kombination mit einer Trägermatte schützt die Trittfläche vor Verschmutzungen und Beschädigungen durch einen Untergrund, auf dem die Vorrichtung verwendet wird. Die Trägermatte kann die gleichen Abmaße und die gleiche Form wie die Trittfläche aufweisen. In weiteren Ausführungen kann die Trägermatte auch größer als die Trittfläche sein. So kann beispielsweise die Trittfläche eine unregelmäßige Form aufweisen, während die Trägermatte eine regelmäßige Form, z. B. eine runde oder eine rechteckige Form, besitzt.

Die Trittfläche kann auch selbst als eine Trägermatte ausgebildet sein, indem die Trittfläche einen mehrschichtigen Aufbau aufweist. Eine unterste Schicht eines solchen mehrschichtigen Aufbaus ist dabei vorteilhaft eine strapazierfähige, schmutzabweisende Schicht, z. B. aus einem Elastomer wie Gummi oder einem Kunststoff. Die Trägermatte kann falt- oder rollbar und somit leichter verstaubar und transportabel ausgeführt sein.

In einer weiteren Ausgestaltung der erfindungsgemäßen Vorrichtung kann die Trägermatte aus Teilträgermatten zusammengesetzt sein. Je nach Ausgestaltung der Vorrichtung mit oder ohne Trägermatte kann auch die Trittfläche aus einer Anzahl von Teiltrittflächen zusammengesetzt sein. Dabei kann jede Teiltrittfläche mindestens einer Teilträgermatte zugeordnet sein. Jede Teiltrittfläche kann eine Oberflächenstruktur aufweisen.

Die erfindungsgemäße Vorrichtung erlaubt ein Begehen und Ertasten der Wegstrecke des Fußparcours. Dabei erfolgt die Sinneswahrnehmung durch sehende Benutzer vorrangig und durch nicht-sehende Benutzer ausschließlich durch den Tastsinn, also mittels haptischer Wahrnehmung. Eine Bewegung des nicht-sehenden Benutzers ist dabei auch ohne zusätzliche auditive oder visuelle Hilfe möglich.

Die Erfindung wird nachfolgend anhand von Ausführungsbeispielen und Abbildungen näher beschrieben. Dabei zeigen
- Fig. 1: ein erstes Ausführungsbeispiel einer erfindungsgemäßen Vorrichtung,
- Fig. 2: ein zweites Ausführungsbeispiel einer erfindungsgemäßen Vorrichtung und
- Fig. 3: ein drittes Ausführungsbeispiel einer erfindungsgemäßen Vorrichtung mit Teiltrittflächen auf Teilträgermatten.

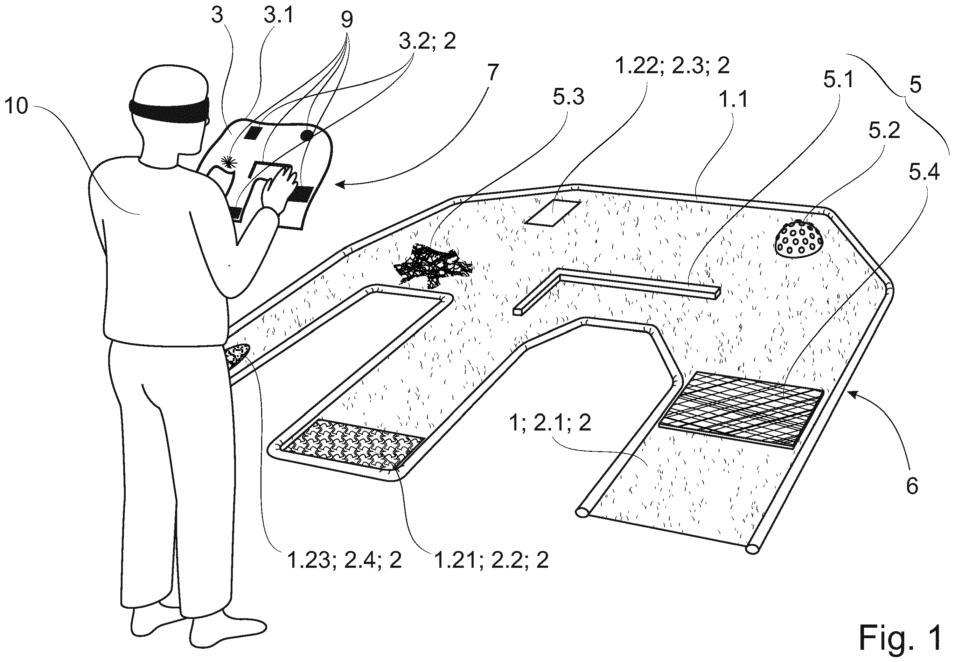

Als wesentliche Elemente eines ersten Ausführungsbeispiels der erfindungsgemäßen Vorrichtung sind in Fig. 1 eine Trittfläche 1 und eine handhaltbare Tastfläche 3 gezeigt, die zueinander im oben genannten geometrischen Sinne ähnlich sind und die jeweils mindestens eine Oberflächenstruktur 2 aufweisen.

Auf der Trittfläche 1, die eine erste Oberflächenstruktur 2.1 aufweist, sind ein erster Trittflächenbereich 1.21 mit einer zweiten Oberflächenstruktur 2.2, ein zweiter Trittflächenbereich 1.22 mit einer dritten Oberflächenstruktur 2.3 und ein dritter Trittflächenbereich 1.23 mit einer vierten Oberflächenstruktur 2.4 vorhanden. Die Trittflächenbereiche 1.21, 1.22, 1.23 sind in die Trittfläche 1 integriert, so dass deren jeweilige Oberflächen in einer Ebene mit der Oberfläche der Trittfläche 1 liegen. Auf der Trittfläche 1 sind als ein erstes Objekt 5.1 ein Winkelelement, als ein zweites Objekt 5.2 eine genoppte Halbschale aus Kunststoff, als ein drittes Objekt 5.3 einige Strohhalme und als ein viertes Objekt 5.4 ein flaches Brett angeordnet. Jedes der Objekte 5 hat eine tatsächliche Objektposition 6 (nur für das vierte Objekt 5.4 gezeigt) relativ zur Trittfläche 1. Die Trittfläche 1 ist durch einen gewölbten Rand begrenzt, dessen Umriss in stilisierter Form einen Elefanten darstellt. Für Trittflächenbereiche 1.2 und Tastflächenbereiche 3.2 gilt das für die tatsächliche Objektposition 6 und eine korrespondierende Objektposition 7 Gesagte entsprechend.

Ein dargestellter Benutzer 10 der erfindungsgemäßen Vorrichtung hält eine Tastfläche 3 in der einen Hand, während er mit der anderen Hand über den Tastflächenbereich 3.2 der Tastfläche 3 streicht. Der Tastflächenbereich 3.2 ist an der korrespondierenden Objektposition 7 auf der Tastfläche 3 angeordnet, die hinsichtlich ihrer Lage auf der Tastfläche 3 der tatsächlichen Objektposition 6 des vierten Objektes 5.4 entspricht. Auf der Tastfläche 3 sind entsprechend der auf der Trittfläche 1 vorhandenen Trittflächenbereiche 1.2 und der angeordneten Objekte 5 Tastflächenbereiche 3.2 (diese vereinfachend mit dem gleichen Bezugszeichen 3.2 versehen) und Tastobjekte 9 angeordnet. Die Tastfläche 3 ist hinsichtlich ihres äußeren Umrisses ähnlich zur Trittfläche 1. Sowohl die Tastflächenbereiche 3.2 als auch die Tastobjekte 9 entsprechen hinsichtlich ihrer Materialbeschaffenheit, Form und Ausdehnung den Trittflächenbereichen 1.2 und Objekten 5 auf der Trittfläche 1. Für die tatsächlichen Objektpositionen 6 und die korrespondierenden Objektpositionen 7 gilt das vorstehend zum vierten Objekt 5.4 Gesagte entsprechend. Die Tastfläche 3 ist aus einem steifen Material gefertigt.

Im gezeigten Ausführungsbeispiel wäre nun der Benutzer 10 auch mit verbundenen Augen (angedeutet) in der Lage, sich auf der Trittfläche 1 zu orientieren, indem er mit dem Fuß erfühlte Umrisse der Trittfläche 1.1 sowie die tatsächlichen Objektpositionen 6, die Ausdehnung und Beschaffenheit der erfühlten Trittflächenbereiche 1.2 und / oder Objekte 5 mit ertasteten Umrissen der Tastfläche 3.1 sowie mit der Ausdehnung und Beschaffenheit der erfühlten Tastflächenbereiche 3.2 und / oder Tastobjekte 9 vergleicht.

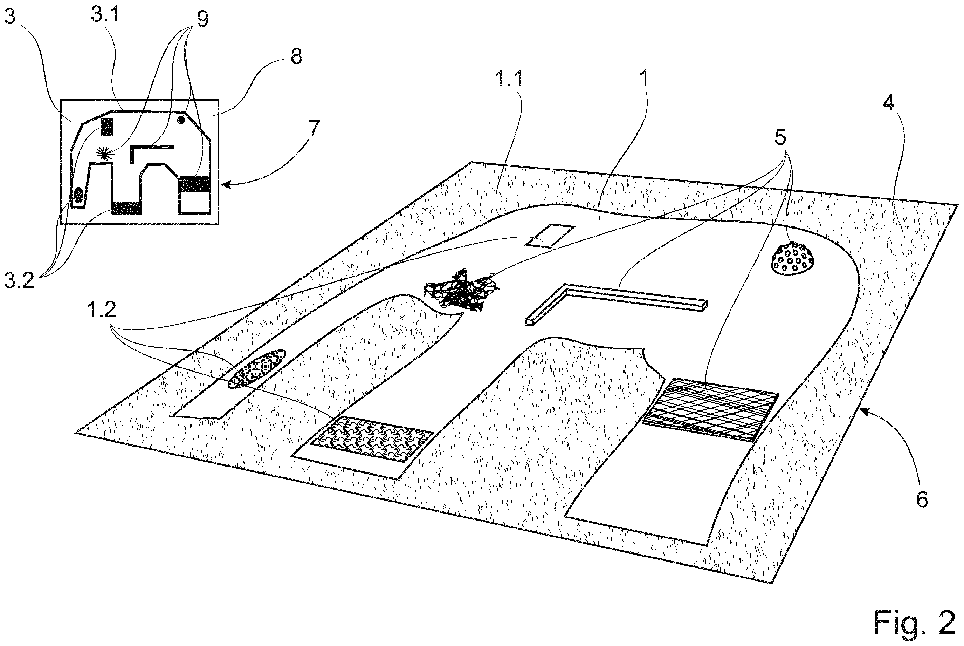

In einem in Fig. 2 dargestellten zweiten Ausführungsbeispiel ist die Trittfläche 1 auf einer rechteckigen Trägermatte 4 vorhanden. Die Trägermatte 4 ragt an allen Seiten über die Umrisse der Trittfläche 1.1 hinaus. Ansonsten entspricht die Trittfläche 1 dem zu Fig. 1 beschriebenen ersten Ausführungsbeispiel. Die handhaltbare Tastfläche 3 ist auf einem rechteckigen Träger 8 aus einem steifen Material angeordnet, der allseitig über die Umrisse der Tastfläche 3.1 ragt. Während die Umrisse der Trittfläche 1.1 einen fließenden Linienverlauf aufweisen, sind die Umrisse der Tastfläche 3.1 vereinfacht dargestellt und weisen einen abschnittsweise geradlinigen Verlauf mit markanten Ecken auf. Auch hier sind Trittfläche 1 und Tastfläche 3 einander ähnlich.

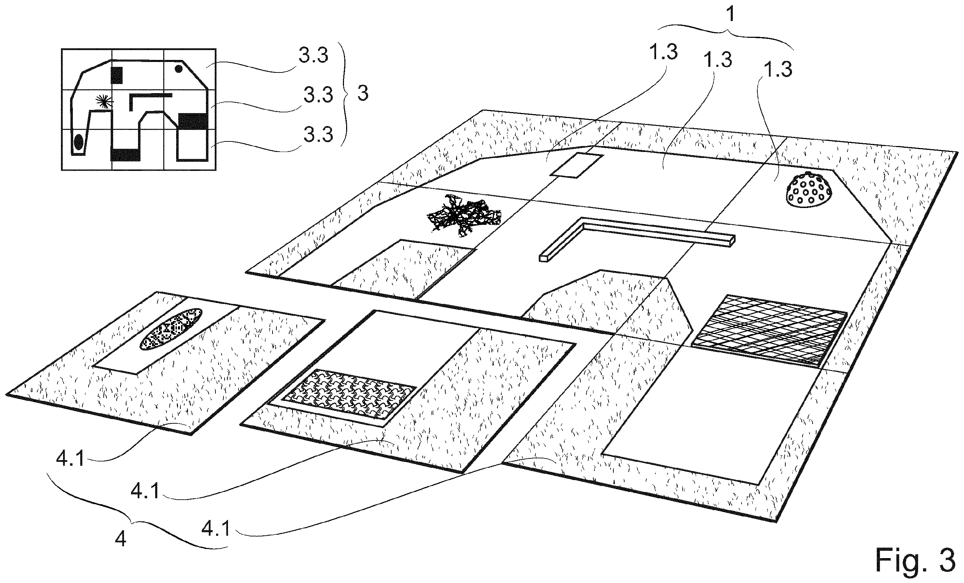

Eine gut zu transportierende Trittfläche 1 ist als drittes Ausführungsbeispiel in Fig. 3 gezeigt. Die Trittfläche 1 ist aus neun Teiltrittflächen 1.3 zusammengesetzt. Zwei der Teiltrittflächen 1.3 sind zur besseren Illustration etwas abgerückt dargestellt. Den Teiltrittflächen 1.3 sind auch Teilträgermatten 4.1 zugeordnet. Die Tastfläche 3 entspricht der zu Fig. 2 beschriebenen, mit dem Unterschied, dass die Tastfläche 3 aus neun Teiltastflächen 3.3 zusammengesetzt ist.

### Bezugszeichenliste

- 1: Trittfläche
- 1.1: Umriss der Trittfläche
- 1.2: Trittflächenbereich
- 1.21: erster Trittflächenbereich
- 1.22: zweiter Trittflächenbereich
- 1.23: dritter Trittflächenbereich
- 1.3: Teiltrittfläche
- 2: Oberflächenstruktur
- 2.1: erste Oberflächenstruktur
- 2.2: zweite Oberflächenstruktur
- 2.3: dritte Oberflächenstruktur
- 2.4: vierte Oberflächenstruktur
- 3: Tastfläche (handhaltbar)
- 3.1: Umriss der Tastfläche
- 3.2: Tastflächenbereich
- 3.3: Teiltastfläche
- 4: Trägermatte
- 4.1: Teilträgermatte
- 5: Objekt
- 5.1: erstes Objekt
- 5.2: zweites Objekt
- 5.3: drittes Objekt
- 5.4: viertes Objekt
- 6: tatsächliche Objektposition
- 7: korrespondierende Objektposition
- 8: Träger (der Tastfläche 3)
- 9: Tastobjekt
- 10: Benutzer

## Patentansprüche

1. Vorrichtung, die eine begehbare Trittfläche (1) und eine handhaltbare Tastfläche (3) aufweist, zur sensorischen Vermittlung von Umwelteindrücken an einen Benutzer (10) und zur Ermittlung eines aktuellen Standorts des Benutzers (10) auf der begehbaren Trittfläche (1) durch Abgleichen von über die Fußsohlen des Benutzers (10) erspürten Sinneseindrücken von der Trittfläche (1) mit den Sinneseindrücken, die der Benutzer (10) über die Hände von der handhaltbaren Tastfläche (3) erhält, wobei
- die Tastfläche (3) zur Trittfläche (1) im geometrischen Sinne ähnlich ist,
- die begehbare Trittfläche (1) Trittflächenbereiche (1.21, 1.22, 1.23) mit jeweils einer Oberflächenstruktur (2.2, 2.3, 2.4) aufweist,
- die handhaltbare Tastfläche (3) mindestens zwei Tastflächenbereiche (3.2) mit jeweils einer Oberflächenstruktur (2) aufweist und
- die Oberflächenstrukturen (2) der Tastflächenbereiche (3.2) den Oberflächenstrukturen (2.2, 2.3, 2.4) der Trittflächenbereiche (1.21, 1.22, 1.23) entsprechen und die Tastflächenbereiche (3.2) hinsichtlich ihrer Lage und Ausdehnung mit den Lagen und Ausdehnungen der Trittflächenbereiche (1.21, 1.22, 1.23) korrespondieren.

2. Vorrichtung nach Anspruch 1, **dadurch gekennzeichnet, dass** auf der Tastfläche (3) eine Anzahl von Tastobjekten (9) an jeweiligen Positionen angeordnet sind.

3. Vorrichtung nach einem der vorhergehenden Ansprüche, **dadurch gekennzeichnet, dass** auf der Trittfläche (1) eine Anzahl von Objekten (5) angeordnet sind, deren jeweilige tatsächliche Objektpositionen (6) bekannt sind.

4. Vorrichtung nach Anspruch 1 bis 3, **dadurch gekennzeichnet, dass** die Positionen der Tastobjekte (9) auf der Tastfläche (3) korrespondierende Objektpositionen (7) sind, die mit den tatsächlichen Objektpositionen (6) der Objekte (5) auf der Trittfläche (1) korrespondieren.

5. Vorrichtung nach Anspruch 4, **dadurch gekennzeichnet, dass** die Tastobjekte (9) auf der Tastfläche (3) den Objekten (5) auf der Trittfläche (1) entsprechen.

6. Vorrichtung nach Anspruch 1, **dadurch gekennzeichnet, dass** die Trittfläche (1) auf einer Trägermatte (4) angeordnet ist.

7. Vorrichtung nach Anspruch 6, **dadurch gekennzeichnet, dass** die Trägermatte (4) aus Teilträgermatten (4.1) zusammengesetzt ist.

## Claims

1. Device, having a walkable tread surface (1) and a hand-held touch surface (3), for sensory transmission of environmental impressions to a user (10) and for determining a current location of the user (10) on the walkable tread surface (1) by matching sensory impressions obtained from the tread surface (1) sensed via the soles of foot of the user (10) with the sensory impressions which the user (10) obtains from the hand-held touch surface (3) via the hands, wherein
- the touch surface (3) is similar to the tread surface (1) in the geometric sense,
- the walkable tread surface (1) has tread surface areas (1.21, 1.22, 1.23) with a surface structure (2.2, 2.3, 2.4) each,
- the hand-held touch surface (3) has at least two touch surface areas (3.2) with a surface structure (2) each and
- the surface structures (2) of the touch surface areas (3.2) correspond with the surface structures (2.2, 2.3, 2.4) of the tread surface areas (1.21, 1.22, 1.23) and the touch surface areas (3.2), with respect to their position and dimension, correspond with the positions and dimensions of the tread surface areas (1.21, 1.22, 1.23).

2. Device according to claim 1, **characterized in that** a number of touch objects (9) are arranged on the touch surface (3) at respective positions.

3. Device according to one of the preceding claims, **characterized in that** a number of objects (5), the respective actual object positions (6) of which are known, are arranged on the tread surface (1).

4. Device according to claim 1 to 3, **characterized in that** the positions of the touch objects (9) on the touch surface (3) are corresponding object positions (7), which correspond with the actual object positions (6) of the objects (5) on the tread surface (1).

5. Device according to claim 4, **characterized in that** the touch objects (9) on the touch surface (3) correspond with the objects (5) on the tread surface (1).

6. Device according to claim 1, **characterized in that** the tread surface (1) is arranged on a carrier mat (4).

7. Device according to claim 6, **characterized in that** the carrier mat (4) is composed of partial carrier mats (4.1).

## Revendications

1. Dispositif, ayant une surface de marche (1) praticable et une surface tactile (3) tenue à la main, de transmission sensorielle d'impressions de l'environnement à un utilisateur (10) et de détermination d'un emplacement actuel de l'utilisateur (10) sur la surface de marche (1) praticable en alignant des impressions sensorielles obtenues de la surface de marche (1) senties via les plantes du pied de l'utilisateur (10) avec les impressions sensorielles que l'utilisateur (10) obtient de la surface tactile (3) tenue à la main via les mains, dans lequel
- la surface tactile (3) est similaire à la surface de marche (1) au sens géométrique,
- la surface de marche (1) praticable a des secteurs de surface de marche (1.21, 1.22, 1.23), chacun avec une structure de surface (2.2, 2.3, 2.4),
- la surface tactile (3) tenue à la main a au moins deux secteurs de surface tactile (3.2), chacun avec une structure de surface (2) et
- les structures de surface (2) des secteurs de surface tactile (3.2) correspondent aux structures de surface (2.2, 2.3, 2.4) des secteurs de surface de marche (1.21, 1.22, 1.23) et les secteurs de surface tactile (3.2), en ce qui concerne leur position et étendue, correspondent aux positions et étendues des secteurs de surface de marche (1.21, 1.22, 1.23).

2. Dispositif selon la revendication 1, **caractérisé en ce qu'**un nombre d'objets tactiles (9) sont disposés sur la surface tactile (3) à des positions respectives.

3. Dispositif selon une des revendications précédentes, **caractérisé en ce qu'**un nombre d'objets (5), dont les positions d'objet effectives (6) respectives sont connues, sont disposés sur la surface de marche (1).

4. Dispositif selon la revendication 1 à 3, **caractérisé en ce que** les positions des objets tactiles (9) sur la surface tactile (3) sont des positions d'objet correspondantes (7) qui correspondent aux positions d'objet effectives (6) des objets (5) sur la surface de marche (1).

5. Dispositif selon la revendication 4, **caractérisé en ce que** les objets tactiles (9) sur la surface tactile (3) correspondent aux objets (5) sur la surface de marche (1).

6. Dispositif selon la revendication 1, **caractérisé en ce que** la surface de marche (1) est disposée sur une natte de support (4).

7. Dispositif selon la revendication 6, **caractérisé en ce que** la natte de support (4) est composée de nattes de support partielles (4.1).
